(19) Europäisches Patentamt
European Patent Office
Office européen des brevets

(11) Numéro de publication : **0 511 073 A1**

# (12) DEMANDE DE BREVET EUROPEEN

(21) Numéro de dépôt : **92401109.1**

(22) Date de dépôt : **21.04.92**

(51) Int. Cl.[5] : **C07F 7/08,** C07D 217/24, C07D 239/70, C07D 275/06, A61K 31/44, A61K 31/695, C07D 211/08, C07D 211/18, C07D 211/70, C07D 279/08, C07D 295/13

(30) Priorité : **26.04.91 FR 9105170**

(43) Date de publication de la demande : **28.10.92 Bulletin 92/44**

(84) Etats contractants désignés : **PT**

(71) Demandeur : **RHONE-POULENC RORER SA
20, avenue Raymond Aron
F-92160 Antony (FR)**

(72) Inventeur : **Damour, Dominique
18 rue Henri Régnault
F-75014 Paris (FR)**
Inventeur : **Labaudinière, Richard
1 place de Valois
F-94220 Charenton (FR)**
Inventeur : **Malleron, Jean-Luc
2 allée Renoir
F-91460 Marcoussis (FR)**
Inventeur : **Mignani, Serge
129 avenue du Général Leclerc
F-75014 Paris (FR)**

(74) Mandataire : **Savina, Jacques et al
Rhône-Poulenc Rorer S.A. Direction Brevets (t144) 20 avenue Raymond Aron
F-92165 Antony Cédex (FR)**

## (54) Antisérotonines, leur préparation et les médicaments les contenant.

(57) Composes de formule :

$$R_1\text{-}(CH_2)_n\text{-Het} \qquad (I)$$

dans laquelle
— $R_1$ représente un reste de formule :

EP 0 511 073 A1

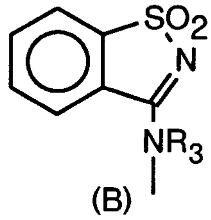

(B)

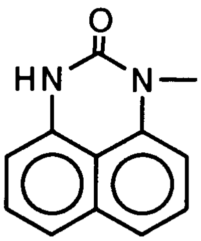

(C)

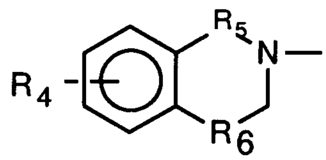

(D)

— Het représente

. un radical phényl-4 tétrahydro-1,2,3,6 pyridyl-1 dont le cycle phényle est éventuellement substitué par un atome d'halogène ou un radical alkyle, alcoxy ou hydroxy,

. un radical phényl-4 pipéridino dont le cycle phényle est éventuellement substitué par un atome d'halogène ou un radical alkyle, alcoxy ou hydroxy,

. un radical phényl-4 pipérazinyl-1 dont le cycle phényle est éventuellement substitué par un atome d'halogène ou un radical alkyle, alcoxy ou hydroxy,

— $R_3$ représente un atome d'hydrogène ou un radical phényle

— $R_4$ représente un atome d'hydrogène ou d'halogène ou un reste Het,

— $R_5$ représente un radical carbonyle ou sulfonyle,

— $R_6$ représente un radical $Si(CH_3)_2$ ou $C(CH_3)_2$

— n est égal à 1,2,3 ou 4,

à l'exception du {[(phényl-4 tétrahydro-1,2,3,6 pyridyl-1)-2 éthyle amino}-3 benzisothiazole-1,2 dioxyde-1,1,

leurs sels, leur préparation et les médicaments les contenant.

La présente invention concerne des composés de formule:

$$R_1\text{-}(CH_2)_n\text{-Het} \qquad (I)$$

leur préparation et les médicaments les contenant.

Des dérivés d'amino-3 benzisothiazole-1,2 dioxyde-1,1 sont décrits dans le brevet US 4104387 comme antiinflammatoires.

Dans la formule (I),

- $R_1$ représente un reste de formule

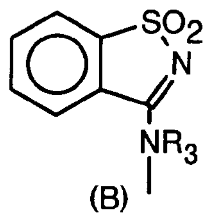

(B)

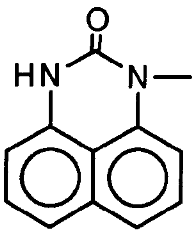

(C)

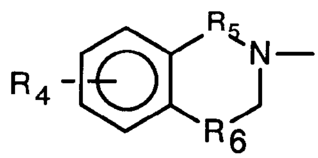

(D)

- Het représente

un radical phényl-4 tétrahydro-1,2,3,6 pyridyl-1 dont le cycle phényle est éventuellement substitué par un atome d'halogène ou un radical alkyle, alcoxy ou hydroxy,

un radical phényl-4 pipéridino dont le cycle phényle est éventuellement substitué par un atome d'halogène ou un radical alkyle, alcoxy ou hydroxy,

un radical phényl-4 pipérazinyl-1 dont le cycle phényle est éventuellement substitué par un atome d'halogène ou un radical alkyle, alcoxy ou hydroxy,

- $R_3$ représente un atome d'hydrogène ou un radical phényle
- $R_4$ représente un atome d'hydrogène ou d'halogène ou un reste Het,
- $R_5$ représente un radical carbonyle ou sulfonyle,
- $R_5$ représente un radical $Si(CH_3)_2$ ou $C(CH_3)_2$,
- n est égal à 1, 2, 3 ou 4,

à l'exception du {[(phényl-4 tétrahydro-1,2,3,6 pyridyl-1)-2 éthyl] amino}-3 benzisothiazole-1,2 dioxyde-1,1.

L'invention concerne également les sels des composés de formule (I) avec les acides minéraux ou organiques.

Dans les définitions qui précèdent et celles qui seront citées ci-après, les radicaux alkyle et alcoxy contiennent 1 à 4 atomes de carbone en chaîne droite ou ramifiée et les atomes d'halogène sont de préférence les atomes de fluor, de chlore ou de brome.

Les composés de formule (I) peuvent être préparés par action d'un dérivé de formule:

$$R_1\text{-}(CH_2)_n\text{-Hal} \qquad (II)$$

dans laquelle $R_1$ et n ont les mêmes significations que dans la formule (I) et Hal représente un atome d'halogène, sur une amine de formule:

$$\text{Het-H} \qquad (III)$$

dans laquelle Het a les mêmes significations que dans la formule (I).

Cette réaction s'effectue généralement au sein d'un solvant inerte tel que le diméthylformamide, le toluène ou le tétrahydrofuranne, en présence d'une base telle qu'un bicarbonate de métal alcalin ou une trialkylamine, à la température d'ébullition du solvant.

Les dérivés de formule (II) à l'exception de ceux pour lesquels $R_1$ représente un reste de formule (C) peuvent être obtenus par action d'un dérivé de formule :

$$R_1H \qquad (IV)$$

dans laquelle $R_1$ a les mêmes significations que dans la formule (I) sur un dérivé dihalogéné de formule :

$$\text{Hal-}(CH_2)_n\text{-Hal} \qquad (V)$$

dans laquelle Hal représente un atome d'halogène, les 2 atomes d'halogène pouvant être identiques ou différents et n a les mêmes significations que dans la formule (I).

Cette réaction s'effectue dans un solvant inerte tel que le diméthylformamide, le tétrahydrofuranne ou l'acétone, au moyen d'hydrure de sodium ou de n-butyllithium, à une température comprise entre 20°C et la température d'ébullition du solvant.

Les dérivés de formule (II) pour lesquels $R_1$ représente un reste de formule (C) peuvent être obtenus par hydrolyse d'un dérivé de formule:

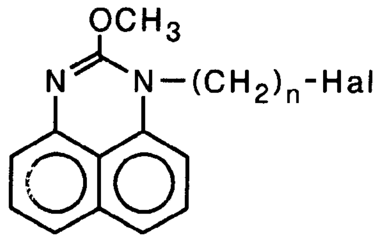

(VI)

dans laquelle Hal représente un atome d'halogène et n a les mêmes significations que dans la formule (I).

Cette hydrolyse s'effectue au moyen d'un acide (acide chlorhydrique par exemple) au sein d'un solvant inerte tel qu'un alcool, l'eau ou un mélange de ces solvants, à une température comprise entre 20°C et la température d'ébullition du solvant.

Les composés de formule (VI) peuvent être préparés par action d'un dérivé dihalogéné de formule (V) sur la méthoxy-2 périmidine.

Cette réaction s'effectue généralement au sein d'un solvant inerte tel que le diméthylformamide, l'acétone ou un mélange de tels solvants, au moyen d'hydrure de sodium, à une température comprise entre 20°C et la température d'ébullition du solvant.

Les composés de formule (IV) sont commercialisés ou peuvent être obtenus par application ou adaptation des méthodes décrites par S. R. SALMAN, J. Chem. Eng. Data, 32, 391 (1987), dans les brevets US 4132725, DE 2438966 et dans les exemples.

Les amines de formule (III) sont commercialisées ou peuvent être obtenues par application ou adaption des méthodes décrites par D. K. YUNK et coll., J. Med. Chem., 21, 1301 (1978); L. THUNUS et coll., Ann. Pharm., 38, 353 (1980); L. GOOTES et coll., Arzneim. Forsch., 17, 1145 (1963) et dans le brevet EP 350403.

Les composés de formule (I) à l'exception de ceux pour lesquels $R_1$ représente un reste de formule (C) peuvent également être préparés par action d'un dérivé de formule (IV) sur un dérivé halogéné de formule:

$$Hal\text{-}(CH_2)_n\text{-}Het \qquad (VII)$$

dans laquelle Hal représente un atome d'halogène, n et Het ont les mêmes significations que dans la formule (I).

Cette réaction s'effectue généralement en présence d'une base telle qu'un hydrure de métal alcalin, un hydroxyde de métal alcalin, un bicarbonate de métal alcalin ou un carbonate de métal alcalin, éventuellement en présence de bromure de tétrabutylammonium, au sein d'un solvant inerte tel que le diméthylformamide ou le tétrahydrofuranne, à une température comprise entre 20°C et la température d'ébullition du solvant.

Les dérivés halogénés de formule (VII) peuvent être obtenus par action d'une amine de formule (III) sur un dérivé dihalogéné de formule (V).

Cette réaction s'effectue généralement au sein d'un solvant inerte tel que le diméthylformamide ou l'acétonitrile, en présence d'une base telle qu'un carbonate de métal alcalin, à une température comprise entre 20°C et la température d'ébullition du solvant.

Les mélanges réactionnels obtenus par les divers procédés décrits précédemment sont traités suivant des méthodes classiques physiques (extraction, évaporation, distillation, chromatographie ...) ou chimiques (formation de sels ...).

Les composés de formule (I) sous forme de base libre peuvent éventuellement être transformés en sels d'addition avec un acide minéral ou organique, par action d'un tel acide au sein d'un solvant organique tel qu'un alcool, une cétone, un éther ou un solvant chloré.

Les composés de formule (I) et leurs sels présentent des propriétés intéressantes. Ces composés possèdent des propriétés antagonistes de la sérotonine (récepteurs 5HT2) et sont donc utiles pour le traitement des affections où la sérotonine est impliquée, notamment les affections du système nerveux central, du système cardiovasculaire et les troubles gastrointestinaux.

Ces composés sont, en particulier, utiles pour le traitement de l'anxiété, des troubles du sommeil, des psychoses et notamment de la schizophrénie, de la migraine, de l'asthme, de l'hypertension et de l'urticaire, comme analgésiques et comme inhibiteurs de l'aggrégation plaquettaire.

L'affinité des composés de formule (I) pour les sites récepteurs centraux à sérotonine (type S2) a été déterminée selon une technique inspirée de celle de J. E. LEYSEN et coll., Mol. Pharmacol., 21, 301 (1982) qui consiste à mesurer l'affinité des produits pour les sites de liaison de la kétansérine tritiée. Dans ce test, la $CI_{50}$ des composés de formule (I) est généralement inférieure à 50nM.

Les composés de formule (I) présentent un toxicité faible. Ils sont généralement atoxiques à 300 mg/kg par voie orale chez la souris en administration unique.

Sont particulièrement intéressants les composés pour lesquels $R_1$ représente un reste de formule (D) et Het représente un radical phényl-4 pipérazinyl-1 dont le cycle phényle est éventuellement substitué par un atome d'halogène et notamment fluor ou un radical alkyle ou hydroxy ou un radical phényl-4 tétrahydro-1,2,3,6 pyridyl-1.

Sont d'un intérêt particulier les composés suivants:

- diméthyl-1,1 oxo-4 {(phényl-4 pipérazinyl-1)-3 propyl}-3 tétrahydro-1,2,3,4 benzazasiline-3,1 sous forme de dichlorhydrate,
- [{(méthyl-4 phényl)-4 pipérazinyl-1}-3 propyl]-3 diméthyl-1,1 oxo-4 tétrahydro-1,2,3,4 benzazasiline-3,1,
- diméthyl-1,1 [{(hydroxy-4 phényl)-4 pipérazinyl-1}-3 propyl]-3 oxo-4 tétrahydro-1,2,3,4 benzazasiline-3,1,
- diméthyl-1,1 [(phényl-4 tétrahydro-1,2,3,4 pyridyl-1)-3 propyl]-3 oxo-4 tétrahydro-1,2,3,4 benzazasiline-3,1,
- diméthyl-1,1 fluoro-5 oxo-4 [(phényl-4 pipérazinyl-1)-3 propyl-3]-3 tétrahydro-1,2,3,4 benzazasiline-3,1,
- diméthyl-4,4 [(fluoro-4 phényl)-4 pipérazinyl-1}-3 propyl]-2 tétrahydro1,2,3,4 isoquinoléinone-1.

Pour l'emploi thérapeutique, il peut être fait usage des composés de formule (I) tels quels ou à l'état de sels pharmaceutiquement acceptables.

Comme sels pharmaceutiquement acceptables, peuvent être cités les sels d'addition avec les acides minéraux tels que chlorhydrates, sulfates, nitrates, phosphates ou organiques tels que acétates, propionates, succinates, oxalates, benzoates, fumarates, maléates, méthanesulfonates, iséthionate, théophillineacétates, phénolphtalinates, salicylates, méthylènebis-β oxynaphtoates ou des dérivés substitués de ces dérivés.

Les exemples suivants donnés à titre non limitatif montrent comment l'invention peut être mise en pratique.

**EXEMPLE 1**

1,35 g de (fluoro-4 phényl)-4 pipérazine, 1,84 g de (chloro-3 propyl)-1 périmidinone-2 et 0,66 g de bicarbonate de sodium dans 25 cm3 de N,N-diméthylformamide sec sont agités au reflux du solvant pendant 2 heures. Le mélange réactionnel est refroidi et versé dans un mélange de 20 cm3 d'eau et 25 cm de dichlorométhane. La phase organique est décantée, lavée à l'eau (3 fois 15 cm3), séchée sur sulfate de magnésium et concentrée à sec sous pression réduite (2,7 kPa). Le résidu est chromatographié sur une colonne de gel de silice sous une pression de 0,7 bar d'azote en éluant par de l'acétate d'éthyle. On obtient ainsi 0,65 g de [[(fluoro-4 phényl)-4 pipérazinyl-1]-3 propyl]-1 périmidinone-2 fondant à 206°C.

La (chloro-3 propyl)-1 périmidinone-2 peut être préparée de la manière suivante : 35 g de (chloro-3 propyl)-3 méthoxy-2 périmidine, 70 cm3 d'acide chlorhydrique concentré, 210 cm3 d'éthanol et 140 cm3 d'eau sont portés à ébullition pendant 10 minutes. Le milieu réactionnel est refroidi à une température proche de 20°C puis le précipité est filtré sur verre fritté et séché. On obtient ainsi 32 g de (chloro-3 propyl)-1 périmidinone-2 fondant à 170°C.

La (chloro-3 propyl)-3 méthoxy-2 périmidine peut être préparée de la manière suivante : 49 g de méthoxy-2 périmidine et 26,7 cm3 de bromo-1 chloro-3 propane dans 420 cm3 d'acétone et 42 cm3 de N,N-diméthylformamide sont portés à ébullition pendant 14 heures. Le milieu réactionnel est refroidi à une température proche de 20°C puis le précipité est filtré sur verre fritté et rincé par 50 cm3 d'oxyde de diéthyle. Le filtrat est concentré à sec sous pression réduite (2,7 kPa). L'huile obtenue est purifiée par chromatographie sur colonne de gel de silice sous une pression de 0,7 bar d'azote en éluant par du toluène. On obtient 34,9 g de (chloro-3 propyl)-3 méthoxy-2 périmidine (Rf 0,7- plaque de silice-toluène).

La méthoxy-2 périmidine peut être préparée de la manière suivante: 50 g de diamino-1,8 naphtalène et 42 g d'orthocarbonate de méthyle sont chauffés à reflux pendant 24 heures. Le milieu réactionnel est refroidi à une température proche de 20°C puis concentré à sec sous pression réduite (2,7 kPa). Le résidu est purifié par chromatographie sur colonne de gel de silice avec du dichlorométhane comme éluant. Après lavage par de l'hexane, on obtient 39,5 g de méthoxy-2 périmidine sous forme de cristaux utilisés tels quels dans les synthèses ultérieures.

**Exemple 2**

On porte 24 heures au reflux un mélange de 2,2 g d'anilino-3 benzisothiazole-1,2 dioxyde-1,1, 2,1 g de (chloro-3 propyl)-1 (fluoro-4 phényl)-4 pipérazine, 3,4 g de carbonate de potassium, 1,1 g de bromure de tétrabutyl ammonium et 25 cm3 de diméthylformamide. Le mélange est ensuite refroidi à une température voisine de 20°C. Le filtrat est évaporé à sec à 40°C sous pression réduite (10 mm de mercure; 1,35 kPa). Le résidu est repris par 20 cm3 d'eau et extrait par 75 cm3 de dichlorométhane. La phase organique ainsi obtenue est séchée sur sulfate de magnésium anhydre et évaporée à sec à 40°C sous pression réduite (20 mm de mercure; 2,7 kPa). L'huile obtenue est purifiée par flash-chromatographie sur colonne de silice, sous courant d'azote à moyenne pression (0,1-1,5 bar) avec de l'acétate d'éthyle comme éluant. On obtient 1,0 g d'une meringue beige qui recristallisée dans 10 cm3 d'éthanol chaud conduit à 0,95 g de {{[(fluoro-4 phényl)-4 pipérazinyl-1]-3 propyl} phényl} amino-3 benzisothiazole-1,2 dioxyde-1,1 fondant à 134°C.

L'anilino-3 benzisothiazole-1,2 dioxyde-1,1 peut être préparée selon la méthode décrite par S.R. SALMAN, J.Chem. Eng. Data, 32, 391, 1987.

**Exemple 3**

On porte 11 heures à 60°C un mélange de 0,55 g d'amino-3 benzisothiazole-1,2 dioxyde-1,1, 0,76 g de (chloro-3 propyl)-1 (fluoro-4 phényl)-4 pipérazine, 0,41 g de carbonate de potassium et 10 cm3 de diméthylformamide. Le mélange est ensuite refroidi à une température voisine de 20°C. Le filtrat est évaporé à sec à 40°C sous pression réduite (10 mm de mercure; 1,35 kPa). Le résidu est repris par 10 cm3 d'eau et extrait par trois fois 10 cm3 de dichlorométhane. La phase organique ainsi obtenue est séchée sur sulfate de magnésium anhydre et évaporée à sec à 40°C sous pression réduite (20 mm de mercure; 2,7 kPa). Le solide jaune obtenu recristallisé dans 10 cm3 d'acétonitrile chaud conduit à 0,85 g de {[(fluoro-4 phényl)-4 pipérazinyl-1]-3 propyl}amino-3 benzisothiazole-1,2 dioxyde-1,1 fondant à 210°C.

L'amino-3 benzisothiazole-1,2 dioxyde-1,1 peut être préparée selon la méthode décrite par S.R. SALMAN, J.Chem. Eng. Data, 32, 391, 1987.

**Exemple 4**

5,34 g de (chloro-3 propyl)-3 diméthyl-1,1 oxo-4 tétrahydro-1,2,3,4 benzazasiline-3,1, 6 cm3 de triéthylamine et 6,5 g de phényl-1 pipérazine dans 10 cm3 de toluène sont chauffés pendant 18 heures à ébullition. Le milieu est ensuite ramené à une température voisine de 20°C, repris par 100 cm3 d'eau et extrait par trois fois 50 cm3 de dichlorométhane. Les phases organiques réunies sont séchées sur sulfate de magnésium et amenées à sec à 40°C sous pression réduite (20 mm de mercure; 2,7 KPa). Le résidu est purifié par flash-chromatographie sur colonne de silice, sous courant d'azote, à moyenne pression (0,5-1,5 bar) avec un mélange dichlorométhaneméthanol (90-10 en volumes) comme éluant. On obtient 6 g d'une huile orangée qui dissoute dans 30 cm3 d'éther diéthylique et addition d'éther chlorhydrique (4N) conduit à 3,4 g de diméthyl-1,1 oxo-4 {(phényl-4 pipérazinyl-1)-3 propyl}-3 tétrahydro-1,2,3,4 benzazasiline-3,1 sous forme de dichlorhydrate fondant à 170°C.

Le (chloro-3 propyl)-3 diméthyl-1,1 oxo-4 tétrahydro-1,2,3,4 benzazasiline-3,1 peut être préparé de la manière suivante : à une solution de 19,1 g de diméthyl-1,1 oxo-4 tétrahydro-1,2,3,4 benzazasiline-3,1 dans 300 cm3 de tétrahydrofuranne, à -76°C, on additionne 75 cm3 de n-butyl lithium (1,6 M dans l'hexane), sous courant d'azote. Le milieu réactionnel est laissé trois heures à 0°C. On ajoute ensuite 37,3 g de chloro-1 bromo-3 propane et le mélange est agité 48 heures à une température proche de 25 °C. Le mélange réactionnel est traité par 100 cm3 d'eau et extrait par trois fois 50 cm3 de dichlorométhane. Les phases organiques réunies sont séchées sur sulfate de magnésium et amenées à sec à 40°C sous pression réduite (20 mm de mercure; 2,7 KPa). Le résidu obtenu est purifié par flash-chromatographie sur colonne de silice, sous courant d'azote, à moyenne pression (0,5-1,5 bar), avec du dichlorométhane comme éluant. On obtient 21 g de (chloro-3 propyl)-3 diméthyl-1,1 oxo-4 tétrahydro-1,2,3,4 benzazasiline-3,1 sous forme d'huile utilisée telle quelle dans les synthèses ultérieures.

Le diméthyl-1,1 oxo-4 tétrahydro-1,2,3,4 benzazasiline-3,1 peut être obtenu selon le procédé décrit dans le brevet US 4132725.

**Exemple 5**

On opère comme dans l'exemple 4, à partir de 2,7 g de (chloro-3 propyl)-3 diméthyl-1,1 oxo-4 tétrahydro-1,2,3,4 benzazasiline-3,1, de 4,3 g de (fluoro-4 phényl)-1 pipérazine et de 3,4 cm3 de triéthylamine dans 50

cm3 de toluène. Le mélange est chauffé 48 heures à ébullition puis refroidi à une température voisine de 25°C. Le milieu est repris par 100 cm3 d'eau et extrait par 50 cm3 de dichlorométhane. Les phases organiques réunies sont séchées sur sulfate de magnésium et amenées à sec à 40°C sous pression réduite (20 mm de mercure; 2,7 KPa). Le résidu est purifié par flash-chromatographie sur colonne de silice, sous courant d'azote, à moyenne pression (0,5-1,5 bar), avec un mélange dichlorométhane-méthanol (98-2 en volumes) comme éluant. On obtient 4 g d'une huile orangée, qui dissoute dans 50 cm3 d'éther éthylique chlorhydrique (4N) conduit à 3,6 g de solide jaune. Ce solide est recristallisé dans 120 cm3 d'un mélange acétonitrileacétate d'éthyle (50-50 en volumes) bouillant et conduit à 1,4 g de diméthyl-1,1 {(fluoro-4 phényl)-4 pipérazinyl-1}-3 propyl]-3 oxo-4 tétrahydro-1,2,3,4 benzazasiline-3,1 sous forme de dichlorhydrate, fondant à 209°C.

**Exemple 6**

On opère comme dans l'exemple 4, à partir de 2,9 g de (chloro-3 propyl)-3 diméthyl-1,1 oxo-4 tétrahydro-1,2,3,4 benzazasiline-3, 1, de 7,9 g du dichlorhydrate de (méthyl-4 phényl)-4 pipérazine et de 9,3 cm3 de triéthylamine dans 60 cm3 de toluène. Le mélange est chauffé 48 heures à ébullition puis refroidi à une température voisine de 25°C. Le milieu est repris par 100 cm3 d'eau et extrait par trois fois 50 cm3 d'acétate d'éthyle. Les phases organiques réunies sont séchées sur sulfate de magnésium et amenées à sec à 40°C sous pression réduite (20 mm de mercure; 2,7 KPa). Le résidu est purifié par flash-chromatographie sur colonne de silice, sous courant d'azote à moyenne pression ( 0,5-1,5 bar) avec un mélange dichlorométhane-méthanol (97,5-2,5 en volumes) comme éluant. On obtient 2,5 g d'une huile orangée, qui dissoute dans 25 cm3 d'acétone et addition de 0,53 g d'acide oxalique conduit à 2,2 g de [{(méthyl-4 phényl)-4 pipérazinyl-1}-3 propyl]-3 diméthyl-1,1 oxo-4 tétrahydro-1,2,3,4 benzazasiline-3,1 sous forme d'oxalate fondant à 153°C.

**Exemple 7**

On opère comme dans l'exemple 4, à partir de 2,9 g de (chloro-3 propyl)-3 diméthyl-1,1 oxo-4 tétrahydro-1,2,3,4 benzazasiline-3,1, de 7,4 g de dibromhydrate d'(hydroxy-4 phényl)-1 pipérazine et de 6,1 cm3 de triéthylamine dans 60 cm3 de toluène. Le mélange est chauffé 48 heures à ébullition puis refroidi à une température voisine de 25°C. Le milieu est repris par 100 cm3 d'eau et extrait par trois fois 50 cm3 d'acétate d'éthyle. Les phases organiques réunies sont séchées sur sulfate de magnésium et amenées à sec à 40°C sous pression réduite (20 mm de mercure; 2,7 KPa).

Le résidu est purifié par flash-chromatographie sur colonne de silice, sous courant d'azote, à moyenne pression (0,5-1,5 bar) avec un mélange dichlorométhane-méthanol (97,5-2,5 en volumes) comme éluant. Après recristallisation dans 50 $cm^3$ d'acétonitrile bouillant, on obtient 1,8 g de diméthyl-1,1 [{(hydroxy-4 phényl)-4 pipérazinyl-1}-3 propyl]-3 oxo-4 tétrahydro-1,2,3,4 benzazasiline-3,1 fondant à 170°C.

**Exemple 8**

On opère comme dans l'exemple 4, à partir de 2,9 g de (chloro-3 propyl)-3 diméthyl-1,1 oxo-4 tétrahydro-1,2,3,4 benzazasiline-3,1, de 3,7 g de phényl-4 pipéridine et de 3 cm de triéthylamine dans 60 cm de toluène. Le mélange est chauffé 24 heures à ébullition puis refroidi à une température voisine de 25°C. Le milieu est repris par 100 cm3 d'eau et extrait par trois fois 50 cm3 d'acétate d'éthyle. Les phases organiques réunies sont séchées sur sulfate de magnésium et amenées à sec à 40°C sous pression réduite (20 mm de mercure; 2,7 KPa). Le résidu est purifié par flash-chromatographie sur colonne de silice, sous courant d'azote, à moyenne pression (0,5-1,5 bar), avec du dichlorométhane comme éluant. On obtient 3,7 g d'une huile orangée, qui dissoute dans 45 cm3 d'acétone et addition de 0,4 g d'acide oxalique, conduit à 2,9 g de diméthyl-1,1 [(phényl-4 pipéridinyl-1)-3 propyl]-3 oxo-4 tétrahydro-1,2,3,4 benzazasiline-3,1 sous forme d'oxalate fondant à 153°C.

**Exemple 9**

On opère comme dans l'exemple 4, à partir de 2,9 g de (chloro-3 propyl)-3 diméthyl-1,1 oxo-4 tétrahydro-1,2,3,4 benzazasiline-3,1, de 4,3 g de phényl-4 tétrahydro-1,2,3,4 pyridine et de 4,6 cm3 de triéthylamine dans 60 cm de toluène. Le mélange est chauffé 24 heures à ébullition puis refroidi à une température voisine de 25°C . Le milieu est repris par 100 cm3 d'eau et extrait par trois fois 50 cm3 d'acétate d'éthyle. Les phases organiques réunies sont séchées sur sulfate de magnésium et amenées à sec à 40°C sous pression réduite (20 mm de mercure; 2,7 KPa). Le résidu est purifié par flash-chromatographie sur colonne de silice, sous courant d'azote, à moyenne pression (0,5-1,5 bar) avec du dichlorométhane comme éluant. On obtient 1,7 g d'une huile orangée, qui dissoute dans 16 cm3 d'acétone et addition de 0,2 g d'acide oxalique, conduit à 0,8 g de

diméthyl-1,1 [(phényl-4 tétrahydro-1,2,3,4 pyridyl-1)-3 propyl]-3 oxo-4 tétrahydro-1,2,3,4 benzazasiline-3,1 sous forme d'oxalate fondant à 182 °C.

**Exemple 10**

On opère comme dans l'exemple 4, à partir de 2,9 g de (chloro-3 propyl)-3 diméthyl-1,1 oxo-4 tétrahydro-1,2,3,4 benzazasiline-3,1, de 4,7 g de (fluoro-4 phényl)-4 tétrahydro-1,2,3,4 pyridine et de 4,6 cm3 de triéthylamine dans 60 cm3 de toluène. Le mélange est chauffé 24 heures à ébullition puis refroidi à une température voisine de 25°C. Le milieu est repris par 100 cm3 d'eau et extrait par trois fois 50 cm3 d'acétate d'éthyle. Les phases organiques réunies sont séchées sur sulfate de magnésium et amenées à sec à 40°C sous pression réduite (20 mm de mercure; 2,7 KPa). Le résidu est purifié par flash-chromatographie sur colonne de silice, sous courant d'azote, à moyenne pression (0,5-1,5 bar) avec un mélange dichlorométhane-méthanol (97,5-2,5 en volumes) comme éluant. On obtient 2,7 g d'une huile orangée, qui dissoute dans 50 cm3 d'acétone et addition de 0,3 g d'acide oxalique, conduit à 0,9 g de diméthyl-1,1 [{(fluoro-4 phényl)-4 tétrahydro-1,2,3,4 pyridyl-1}-3 propyl]-3 oxo-4 tétrahydro-1,2,3,4 benzazasiline-3,1 sous forme d'oxalate fondant à 130°C.

**Exemple 11**

0,65g de (chloro-3 propyl)-3 diméthyl-1,1 fluoro-7 oxo-4 tétrahydro-1,2,3,4 benzazasiline-3,1, 0,7 cm3 de triéthylamine et 0,74 g de phényl-1 pipérazine dans 20 cm3 de toluène sont chauffés pendant 18 heures à ébullition. Le milieu est ensuite ramené à une température voisine de 20°C, repris par 30 cm3 d'eau et extrait par deux fois 20 cm3 d'acétate d'éthyle. Les phases organiques réunies sont séchées sur sulfate de magnésium et amenées à sec à 40°C sous pression réduite (20 mm de mercure; 2,7 KPa). Le résidu est purifié par flash-chromatographie sur colonne de silice, sous courant d'azote, à moyenne pression (0,5-1,5 bar) avec un mélange dichlorométhane-méthanol (98-2 en volumes) comme éluant. On obtient 0,5 g d'une huile orangée qui dissoute dans 10 cm3 d'acétone et addition de 0,1 g d'acide oxalique conduit au diméthyl-1,1 fluoro-7 oxo-4 [(phényl-4 pipérazinyl-1)-3 propyl]-3 tétrahydro-1,2,3,4 benzazasiline-3,1 sous forme d'oxalate fondant à 155°C.

Le (chloro-3 propyl)-3 diméthyl-1,1 fluoro-7 oxo-4 tétrahydro-1,2,3,4 benzazasiline-3,1 peut être préparé de la manière suivante : une solution de 2,5 g de diméthyl-1,1 fluoro-7 oxo-4 tétrahydro-1,2,3,4 benzazasiline-3,1 dans 40 cm3 de tétrahydrofuranne, à -76°C, on additionne 9 cm3 de n-butyl lithium (1,6 M dans l'hexane), sous courant d'azote. Le milieu réactionnel est laissé trois heures à 0°C. On ajoute ensuite 4,53 g de chloro-1 bromo-3 propane et le mélange est agité 48 heures à une température proche de 25°C. Le mélange réactionnel est traité par 100 cm3 d'eau et extrait par trois fois 25 cm3 d'acétate d'éthyle. Les phases organiques réunies sont séchées sur sulfate de magnésium et amenées à sec à 40°C sous pression réduite (20 mm de mercure; 2,7 KPa). Le résidu obtenu est purifié par flash-chromatographie sur colonne de silice, sous courant d'azote, à moyenne pression (0,5-1,5 bar), avec du dichlorométhane comme éluant. On obtient 0,65 g de (chloro-3 propyl)-3 diméthyl-1,1 fluoro-7 oxo-4 tétrahydro-1,2,3,4 benzazasiline-3,1 sous forme d'huile utilisée telle quelle dans les synthèses ultérieures.

Le diméthyl-1,1 fluoro-7 oxo-4 tétrahydro-1,2,3,4 benzazasiline-3,1 peut être préparé de la manière suivante : à une solution de 27,1 g de N-tert butyl(fluoro-4)benzamide dans 930 cm3 de tétrahydrofuranne, à -76°C , on additionne 260 cm3 de n-butyl lithium (1,6 M dans l'hexane), sous courant d'azote. Le milieu réactionnel est laissé trois heures à 0°C. On ajoute ensuite 39,75 g de diméthyl (chlorométhyl) chlorosilane et le mélange est agité pendant 48 heures à 25°C. Le mélange est traité par 1000 cm3 d'eau et extrait par trois fois 400 cm3 d'acétate d'éthyle. Les phases organiques réunies sont séchées sur sulfate de magnésium et amenées à sec à 40°C sous pression réduite (20 mm de mercure; 2,7 KPa). Le résidu obtenu est traité à 5°C par 8 cm3 d'acide sulfurique (d=1,87) puis agité pendant deux heures à 25°C. Après neutralisation du milieu par de la soude aqueuse (d=1,33) et extraction par trois fois 50 cm3 de dichlorométhane, les phases organiques réunies sont séchées sur sulfate de magnésium et amenées à sec à 40°C sous pression réduite (20 mm de mercure; 2,7 KPa). Le résidu obtenu est purifié par flash-chromatographie sur colonne de silice, sous courant d'azote, à moyenne pression (0,5-1,5 bar) avec un mélange dichlorométhane-méthanol (97,5-2,5 en volumes) comme éluant. On obtient 5,05 g de diméthyl-1,1 fluoro-7 oxo-4 tétrahydro-1,2,3,4 benzazasiline-3,1 fondant à 95°C.

Le N-tert butyl(fluoro-4)benzamide peut être préparé par application de la méthode décrite par M.P. SPRATT et H.C.DORN, Ann. Chem., 56 (12) , 2038 (1984).

**Exemple 12**

On opére comme dans l'exemple 11 à partir de 1,4 g de (chloro-3 propyl)-3 diméthyl-1,1 fluoro-8 oxo-4 tétrahydro-1,2,3,4 benzazasiline-3,1, 1,4 cm3 de triéthylamine et 1,58 g de phényl-1 pipérazine dans 45 cm3 de toluène. Après purification par flash-chromatographie sur colonne de silice, sous courant d'azote, à moyenne pression (0,5-1,5 bar) avec un mélange dichlorométhane-méthanol (98,5-1,5 en volumes) comme éluant, on obtient 1,1 g d'une huile, qui dissoute dans 20 cm3 d'acétone et addition de 0,24 g d'acide oxalique conduit au diméthyl-1,1 fluoro-8 oxo-4 [(phényl-4 pipérazinyl-1)-3 propyl-3]-3 tétrahydro-1,2,3,4 benzazasiline-3,1 sous forme d'oxalate fondant à 187°C.

Le (chloro-3 propyl)-3 diméthyl-1,1 fluoro-8 oxo-4 tétrahydro-1,2,3,4 benzazasiline-3,1 est obtenu d'une manière analogue à celle décrite à l'exemple 4 pour la préparation du (chloro-3 propyl)-3 diméthyl-1,1 oxo-4 tétrahydro-1,2,3,4 benzazasiline-3,1 mais à partir de 4,8 g diméthyl-1,1 fluoro-8 oxo-4 tétrahydro-1,2,3,4 benzazasiline-3,1, de 17,2 cm3 de n-butyl lithium, de 8,7 g de chloro-1 bromo-3 propane et de 70 cm3 de tétrahydrofuranne. Après purification par flash-chromatographie sur colonne de silice, sous courant d'azote, à moyenne pression (0,5-1,5 bar) avec un mélange dichlorométhane-méthanol (98-2 en volumes) comme éluant, on obtient 2,75 g de (chloro-3 propyl)-3 diméthyl-1,1 fluoro-8 oxo-4 tétrahydro-1,2,3,4 benzazasiline-3,1 .

Le diméthyl-1,1 fluoro-8 oxo-4 tétrahydro-1,2,3,4 benzazasiline-3,1 peut être obtenu d'une manière analogue à celle décrite à l'exemple 11 pour la préparation du diméthyl-1,1 fluoro-7 oxo-4 tétrahydro-1,2,3,4 benzazasiline-3,1 mais à partir de 27,7 g de N-tert butyl (fluoro-3) benzamide, de 266 cm3 de n-butyllithium, de 40,6 g de diméthyl(chlorométhyl) chlorosilane et de 950 cm3 de tétrahydrofuranne. Après purification, on obtient 7,7 g d'une huile, qui traitée par 8 cm3 d'acide sulfurique (d=1,87) à 5°C, puis neutralisation par de la soude aqueuse (d=1,33), conduit après purification à 4,8 g de diméthyl-1,1 fluoro-8 oxo-4 tétrahydro-1,2,3,4 benzazasiline-3,1 fondant à 132°C.

Le N-tert butyl (fluoro-3) benzamide est préparé par application de la méthode décrite par M.P. SPRATT- et H.C.DORN, Ann. Chem., 56 (12), 2038, (1984).

**Exemple 13**

On opère comme dans l'exemple 11 à partir de 1,15 g de (chloro-3 propyl)-3 diméthyl-1,1 fluoro-5 oxo-4 tétrahydro-1,2,3,4 benzazasiline-3,1, 1,1 cm3 de triéthylamine et 1,3 g de phényl-1 pipérazine dans 23 cm3 de toluène. Après purification par flash-chromatographie sur colonne de silice, sous courant d'azote, à moyenne pression (0,5-1,5 bar) avec un mélange dichlorométhane-méthanol (98,5-1,5 en volumes) comme éluant, on obtient 0,3 g de diméthyl-1,1 (phényl-4 pipérazinyl-1)-5 [(phényl-4 pipérazinyl-1)-3 propyl]-3 oxo-4 tétrahydro-1,2,3,4 benzazasiline-3,1 fondant à 166°C et 0,3 g d'une huile, qui dissoute dans 3 cm3 d'acétone et traitée par 0,24 g d'acide oxalique conduit au diméthyl-1,1 fluoro-5 oxo-4 [(phényl-4 pipérazinyl-1)-3 propyl-3]-3 tétrahydro-1,2,3,4 benzazasiline-3,1 sous forme d'oxalate fondant à 99°C.

Le (chloro-3 propyl)-3 diméthyl-1,1 fluoro-5 oxo-4 tétrahydro-1,2,3,4 benzazasiline-3,1 est obtenu d'une manière analogue à celle décrite dans l'exemple 11 pour la préparation du (chloro-3 propyl)-3 diméthyl-1,1 fluoro-7 oxo-4 tétrahydro-1,2,3,4 benzazasiline-3,1 mais à partir de 3,15 g de diméthyl-1,1 fluoro-5 oxo-4 tétrahydro-1,2,3,4 benzazasiline-3,1, 11,3 cm3 de n-butyl lithium, 5,7 g de chloro-1 bromo-3 propane et 65 cm de tétrahydrofuranne. Après purification par flash-chromatographie sur colonne de silice, sous courant d'azote, à moyenne pression (0,5-1,5 bar) avec un mélange dichlorométhane-méthanol (99,5-0,5 en volumes) comme éluant, on obtient 1,15 g de (chloro-3 propyl)-3 diméthyl-1,1 fluoro-5 oxo-4 tétrahydro-1,2,3,4 benzazasiline-3,1 .

Le diméthyl-1,1 fluoro-5 oxo-4 tétrahydro-1,2,3,4 benzazasiline-3,1 peut être obtenu d'une manière analogue à celle décrite à l'exemple 11 pour la préparation du diméthyl-1,1 fluoro-7 oxo-4 tétrahydro-1,2,3,4 benzazasiline-3,1 mais à partir de 34,1 g de N-tert butyl (fluoro-1) benzamide, 328 cm3 de n-butyllithium, 50 g de diméthyl(chlorométhyl) chlorosilane et 1170 cm3 de tétrahydrofuranne. Après purification, on obtient 4,4 g d'une huile, qui traitée par 10 cm3 d'acide sulfurique (d=1,87) à 5°C, puis neutralisation par de la soude aqueuse (d=1,33), conduit après purification à 3,15 g de diméthyl-1,1 fluoro-5 oxo-4 tétrahydro-1,2,3,4 benzazasiline-3,1 fondant à 133°C.

Le N-tert butyl (fluoro-1) benzamide est préparé par application de la méthode décrite par par M. P. SPRATT et H. C. DORN , Ann. Chem. , 56 (12) , 2038, (1984).

**Exemple 14**

1,25 g de (chloro-3 propyl)-2 diméthyl-4,4 tétrahydro-1,2,3,4 isoquinoléinone-1, 1,4 cm3 de triéthylamine et 1,62 g de phényl-1 pipérazine dans 25 cm3 de toluène sont chauffés pendant 24 heures à ébullition. Le milieu

réactionnel est ensuite ramené à une température voisine de 25°C, repris par 50 cm3 d'eau et extrait par trois fois 25 cm3 d'acétate d'éthyle. Les phases organiques sont réunies, séchées sur sulfate de magnésium et amenées à sec à 40°C sous pression réduite (20 mm de mercure; 2,7 KPa). Le résidu est purifié par flash-chromatographie sur colonne de silice, sous courant d'azote, à moyenne pression (0,5-1,5 bar) avec un mélange dichlorométhane-méthanol (99-1 en volumes) comme éluant. On obtient 1,6 g d'une huile. orangée, qui dissoute dans 40 cm3 d'acétone et addition de 0,43 g d'acide oxalique conduit à 1,4 g de diméthyl-4,4 [(phényl-4 pipérazinyl-1)-3 propyl]-2 tétrahydro-1,2,3,4 isoquinoléinone-1 sous forme d'oxalate fondant à 140°C.

Le (chloro-3 propyl)-2 diméthyl-4,4 tétrahydro-1,2,3,4 isoquinoléinone-1 peut être obtenu de la façon suivante : à une solution de 6 g de diméthyl-4,4 tétrahydro-1,2,3,4 isoquinoléinone-1 dans 120 cm3 de tétrahydrofuranne, à -76°C, on additionne 25,7 cm3 de n-butyllithium (1,6 M dans l'hexane), sous courant d'azote. Le milieu réactionnel est laissé trois heures à 0°C. On ajoute ensuite 13 g de chloro-1 bromo-3 propane et le mélange est agité 18 heures à une température voisine de 25°C.Le mélange réactionnel est traité par 240 cm3 d'eau et extrait par trois fois 100 cm3 d'acétate d'éthyle. Les phases organiques réunies sont séchées sur sulfate de magnésium et amenées à sec à 40°C sous pression réduite (20 mm de mercure; 2,7 KPa). Le résidu est purifié par flash-chromatographie sur colonne de silice, sous courant d'azote, à moyenne pression (0,5-1,5 bar), avec un mélange dichlorométhane-méthanol (99,5-0,5 en volumes) comme éluant. On obtient 3,9 g de (chloro-3 propyl)-2 diméthyl-4,4 tétrahydro-1,2,3,4 isoquinoléinone-1 fondant à 131 °C.

Le diméthyl-4,4 tétrahydro-1,2,3,4 isoquinoléinone-1 peut être obtenu selon le procédé décrit dans le brevet DE 2438966.

**Exemple 15**

On opère comme dans l'exemple 14, à partir de 2,2 g de (chloro-3 propyl)-2 diméthyl-4,4 tétrahydro-1,2,3,4 isoquinoléinone-1, 2,5 cm3 de triéthylamine et 3,1 g de (fluoro-4 phényl)-1 pipérazine dans 25 cm3 de toluène. Le milieu réactionnel est chauffé à ébullition pendant 24 heures puis ramené à une température voisine de 25°C, repris par 50 cm3 d'eau et extrait par trois fois 25 cm3 d'acétate d'éthyle. Les phases organiques sont réunies, séchées sur sulfate de magnésium et amenées à sec à 40°C sous pression réduite (20 mm de mercure; 2,7 KPa). Le résidu est purifié par flash-chromatographie sur colonne de silice, sous courant d'azote, à moyenne pression (0,5-1,5 bar), avec un mélange dichlorométhane-méthanol (99,5-0,5 en volumes) comme éluant. On obtient 3,2 g d'une huile, qui dissoute dans 20 cm3 d'acétone et addition de 0,7 g d'acide oxalique conduit à 2,85 g de diméthyl-4,4 [(fluoro-4 phényl)-4 pipérazinyl-1}-3 propyl]-2 tétrahydro-1,2,3,4 isoquinoléinone-1 sous forme d'oxalate fondant à 155°C.

**Exemple 16**

On opère comme dans l'exemple 4, à partir de 1,95 g de diméthyl-4,4 (chloro-3 propyl)-2 3H-benzothiazasiline-1,2,4 dioxyde-1,1, de 2 g de (fluoro-4 phényl)-1 pipérazine, de 1,5 cm3 de triéthylamine et de 35 cm3 de toluène. Après purification par flash-chromatographie sur colonne de silice, sous courant d'azote, à moyenne pression (0,5-1,5 bar) avec un mélange dichlorométhane-méthanol (98-2 en volumes) comme éluant, on obtient 2,4 g d'une huile, qui dissoute dans 40 cm3 d'acétone et addition de 0,5 g d'acide oxalique conduit à 1,7 g de diméthyl-4,4 {[(fluoro-4 phényl)-4 pipérazinyl-1]-3 propyl}-2 3H-benzothiazasiline-1,2,4 dioxyde-1,1 sous forme d'oxalate fondant à 153°C.

Le diméthyl-4,4 (chloro-3 propyl)-2 3H-benzothiazasiline-1,2,4 dioxyde-1,1 peut être obtenu d'une manière analogue à celle décrite à l'exemple 4 pour la préparation du (chloro-3 propyl)-3 diméthyl-1,1 oxo-4 tétrahydro-1,2,3,4 benzazasiline-3,1 mais à partir de 3,2 g de diméthyl-4,4 3H-benzothiazasiline-1,2,4 dioxyde-1,1, 10,5 cm3 de n-butyl lithium, 5,3 g de chloro-1 bromo-3 propane et 50 cm3 de tétrahydrofuranne. Après purification par flash-chromatographie sur colonne de silice, sous courant d'azote, à moyenne pression (0,5-1,5 bar), avec un mélange dichlorométhane-cyclohexane (50-50 en volumes) comme éluant, on obtient 1,95 g de diméthyl-4,4 (chloro-3 propyl)-2 3H-benzothiazasiline-1,2,4 dioxyde-1,1.

Le diméthyl-4,4 3H-benzothiazasiline-1,2,4 dioxyde-1,1 peut être préparé de la manière suivante : 4,6 g de N-tert butyl benzènesulfonamide en solution dans 45 cm3 de tétrahydrofuranne sont traités à -76°C par 37,5 cm3 de n-butyllithium. Le milieu réactionnel est agité pendant trois heures à 0°C, puis on ajoute 5,72 g de diméthyl(chlorométhyl)chlorosilane. Après agitation à 25°C pendant 18 heures, on traite le milieu par 100 cm3 d'eau et on extrait par trois fois 75 cm3 d'acétate d'éthyle. Les phases organiques réunies sont séchées sur sulfate de magnésium et amenées à sec à 40°C sous pression réduite (20 mm de mercure; 2,7 KPa). Le résidu est traité par 3 cm3 d'acide sulfurique (d=1,84) à 5°C. Après neutralisation par de la soude aqueuse (d=1,33), extraction par trois fois 50 cm3 de dichlorométhane et purification, on obtient 1,6 g de diméthyl-4,4 3H-benzothiazasiline-1,2,4 dioxyde-1,1 fondant à 130 C.

Le N-tert-butyl benzènesulfoamide peur être préparé selon le procédé décrit dans le brevet EP 333557.

Les médicaments selon l'invention sont constitués par un composé de formule (I) sous forme libre ou sous forme d'un sel d'addition avec un acide pharmaceutiquement acceptable, à l'état pur ou sous forme d'une composition dans laquelle il est associé à tout autre produit pharmaceutiquement compatible, pouvant être inerte ou physiologiquement actif. Les médicaments selon l'invention peuvent être employés par voie orale, parentérale, rectale ou topique.

Comme compositions solides pour administration orale, peuvent être utilisés des comprimés, des pilules, des poudres (capsules de gélatine, cachets) ou des granulés. Dans ces compositions, le principe actif selon l'invention est mélangé à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être des solutions aqueuses ou non aqueuses, des suspensions ou des émulsions. Comme solvant ou véhicule on peut employer l'eau, le propylèneglycol, un polyéthylèneglycol, des huiles végétales, en particulier l'huile d'olive, des esters organiques injectables, par exemple l'oléate d'éthyle ou autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectales qui contiennent, outre le produit actif, des excipients tels que le beurre de cacao, des glycérides semi-synthétiques ou des polyéthylèneglycols.

Les compositions pour administration topique peuvent être par exemple des crèmes, pommades, lotions, collyres, collutoires, gouttes nasales ou aérosols.

En thérapeutique humaine, les composés selon l'invention sont particulièrement utiles pour le traitement des affections ou la sérotonine est impliquée et notamment les affections du système nerveux central, du système cardiovasculaire et les troubles intestinaux. Ils sont en particulier utiles pour le traitement de l'anxiété, des troubles du sommeil, de la dépression, des psychoses et notamment de la schizophrénie, de la migraine, de l'asthme, de l'hypertension et de l'urticaire, comme analgésiques et comme inhibiteurs de l'agrégation plaquettaire.

Les doses dépendent de l'effet recherché et de la voie d'administration utilisée; elles sont généralement comprises entre 10 et 300 mg par jour par voie orale pour un adulte avec des doses unitaires allant de 5 à 150 mg de substance active.

D'une façon générale, le médecin déterminera la posologie appropriée en fonction de l'âge, du poids et de tous les autres facteurs propres au sujet à traiter.

Les exemples suivants illustrant des compositions selon l'invention:

## EXEMPLE A

On prépare, selon la technique habituelle, des gélules dosées à 50 mg de produit actif ayant la composition suivante :

| | |
|---|---|
| - Diméthyl-1,1 fluoro-5 oxo-4 [(phényl-4 pipérazinyl-1)-3 propyl-3]-3 tétrahydro-1,2,3,4 benzazasiline-3,1 .......... | 50 mg |
| - Cellulose ........ | 18 mg |
| - Lactose ........ | 55 mg |
| - Silice colloïdale ........ | 1 mg |
| - Carboxyméthylamidon sodique ........ | 10 mg |
| - Talc ........ | 10 mg |
| - Stéarate de magnésium ........ | 1 mg |

EXEMPLE B

On prépare selon la technique habituelle de comprimés dosés à 50 mg de produit actif ayant la composition suivante :

| | |
|---|---|
| - Diméthyl-1,1 oxo-4 {(phényl-4 pipérazinyl-1)-3 propyl}-3 tétrahydro-1,2,3,4 benzazasiline-3,1 ........ | 50 mg |
| - Lactose ........ | 104 mg |
| - Cellulose ........ | 40 mg |
| - Polyvidone ........ | 10 mg |
| - Carboxyméthylamidon sodique ........ | 22 mg |
| - Talc ........ | 10 mg |
| - Stéarate de magnésium ........ | 2 mg |
| - Silice colloïdale ........ | 2 mg |
| - Mélange d'hydroxyméthylcellulose, glycérine, oxyde de titane (72-3,5-24,5) q.s.p. 1 comprimé pelliculé terminé à | 245 mg |

EXEMPLE C

On prépare une solution injectable contenant 10 mg de produit actif ayant la composition suivante :

| | |
|---|---|
| - Diméthyl-1,1 fluoro-5 oxo-4 {(phényl-4 pipérazinyl-1)-3 propyl}-3 tétrahydro-1,2,3,4 benzazasiline-3,1 ........ | 10 mg |
| - Acide benzoïque ........ | 80 mg |
| - Alcool benzylique ........ | 0,06 cm3 |
| - Benzoate de sodium ........ | 80 mg |
| - Ethanol à 95 % ........ | 0,4 cm3 |
| - Hydroxyde de sodium ........ | 24 mg |
| - Propylène glycol ........ | 1,6 cm3 |
| - Eau ........ q.s.p. | 4 cm3 |

## Revendications

**1 -** Composés de formule:

$$R_1\text{-}(CH_2)_n\text{-Het} \qquad (I)$$

dans laquelle:

- $R_1$ représente un reste de formule:

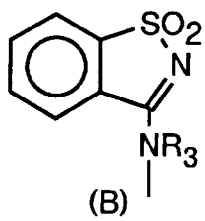

(B)

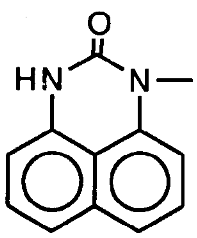

(C)

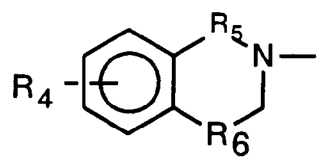

(D)

- Het représente
  . un radical phényl-4 tétrahydro-1,2,3,6 pyridyl-1 dont le cycle phényle est éventuellement substitué par un atome d'halogène ou un radical alkyle, alcoxy ou hydroxy,
  . un radical phényl-4 pipéridino dont le cycle phényle est éventuellement substitué par un atome d'halogène ou un radical alkyle, alcoxy ou hydroxy,
  . un radical phényl-4 pipérazinyl-1 dont le cycle phényle est éventuellement substitué par un atome d'halogène ou un radical alkyle, alcoxy ou hydroxy,
- $R_3$ représente un atome d'hydrogène ou un radical phényle
- $R_4$ représente un atome d'hydrogène ou d'halogène ou un reste Het,
- $R_5$ représente un radical carbonyle ou sulfonyle,
- $R_5$ représente un radical $Si(CH_3)_2$ ou $C(CH_3)_2$,
- n est égal à 1, 2, 3 ou 4.

à l'exception du {[(phényl-4 tétrahydro-1,2,3,6 pyridyl-1)-2 éthyl] amino}-3 benzisothiazole-1,2 dioxyde-1,1 et étant entendu que les radicaux alkyle et alcoxy contiennent 1 à 4 atomes de carbone en chaîne droite ou ramifiée et les sels de ces composés avec un acide organique ou minéral.

**2 -** Composés de formule (I) selon la revendication 1 pour lesquels $R_1$ représente un reste de formule (D) et Het représente un radical phényl-4 pipérazinyl-1 dont le cycle phényle est éventuellement substitué par un atome d'halogène et notamment fluor ou un radical alkyle ou hydroxy ou un radical phényl-4 tétrahydro-1,2,3,6 pyridyl-1.

**3 -** Procédé de préparation des composés de formule (I) selon la revendication 1 caractérisé en ce que l'on fait réagir un dérivé de formule:

$$R_1\text{-}(CH_2)_n\text{-Hal} \qquad (II)$$

dans laquelle $R_1$ et n ont les mêmes significations que dans la revendication 1 et Hal représente un atome d'halogène sur une amine de formule:

$$\text{Het-H} \qquad (III)$$

dans laquelle Het a les mêmes significations que dans la revendication 1, isole le produit et le transforme éventuellement en sel.

**4 -** Procédé de préparation des composés de formule (I) selon la revendication 1 à l'exception de ceux pour lesquels $R_1$ représente un reste de formule (C) , caractérisée en ce que l'on fait réagir un dérivé de formule :

$$R_1H \qquad (IV)$$

dans laquelle $R_1$ a les mêmes significations que ci-dessus sur un dérivé halogéné de formule:

$$Hal-(CH_2)_n-Het \qquad (VII)$$

dans laquelle Hal représente un atome d'halogène, Het et n ont les mêmes significations que dans la revendication 1, isole le produit et le transforme éventuellement en sel.

**5 -** Médicaments caractérisés en ce qu'ils contiennent comme matière active au moins un composé de formule (I) selon la revendication 1.

**6 -** Médicaments caractérisés en ce qu'ils contiennent comme matière active au moins un composé selon la revendication 2.

**7 -** Médicaments selon les revendications 5 ou 6 pour le traitement des maladies où la sérotonine est impliquée.

Office européen des brevets

# RAPPORT DE RECHERCHE EUROPEENNE

Numero de la demande

EP 92 40 1109

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| P,X | JOURNAL OF MEDICINAL CHEMISTRY, vol. 34, no. 8, 1 août 1991, pages 2477-2483; J.-L. MALLERON et al.: "Naphthosultam derivatives: A new class of potent and selective 5-HT2 antagonists<br>* Le document en entier *<br>--- | 1-7 | C 07 F 7/08<br>C 07 D 217/24<br>C 07 D 239/70<br>C 07 D 275/06<br>A 61 K 31/44<br>A 61 K 31/695<br>C 07 D 211/08<br>C 07 D 211/18<br>C 07 D 211/70<br>C 07 D 279/08<br>C 07 D 295/13 |
| A | EP-A-0 330 065 (KOWA CO., LTD)<br>* Le document en entier *<br>--- | 1,5 | |
| A | CH-B- 645 628 (Dr. KARL THOMAE GmbH)<br>* Le document en entier *<br>--- | 1,5 | |
| D,A | US-A-4 132 725 (S. BARCZA)<br>* Le document en entier *<br>--- | 1,5 | |
| A | EP-A-0 329 168 (BRISTOL-MYERS CO.)<br>* Le document en entier *<br>--- | 1,5 | |
| D,A | US-A-4 104 387 (P.C. WADE et al.)<br>* Le document en entier *<br>--- | 1,5 | **DOMAINES TECHNIQUES RECHERCHES (Int. Cl.5)** |
| A | EP-A-0 398 425 (JANSSEN PHARMACEUTICA N.V.)<br>* Le document en entier *<br>--- | 1,5 | C 07 F<br>C 07 D<br>A 61 K |
| A | EP-A-0 269 968 (Dr. KARL THOMAE GmbH)<br>* Le document en entier *<br>--- | 1,5 | |
| A | EP-A-0 089 089 (DUPHAR INTERNATIONAL RESEARCH B.V.)<br>* Le document en entier *<br>--- | 1,5 | |
| A | FR-A-2 154 520 (AB KABI)<br>* Le document en entier *<br>--- -/- | 1,5 | |

Le présent rapport a été établi pour toutes les revendications

| Lieu de la recherche | Date d'achèvement de la recherche | Examinateur |
|---|---|---|
| LA HAYE | 02-07-1992 | RINKEL L.J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.....................................................................
& : membre de la même famille, document correspondant

EPO FORM 1503 03.82 (P0402)

Page 2

Office européen des brevets

# RAPPORT DE RECHERCHE EUROPEENNE

Numero de la demande

EP 92 40 1109

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| P,X | EP-A-0 433 149 (RHONE-POULENC SANTE)<br>* Le document en entier *<br>----- | 1-7 | |
| | | | DOMAINES TECHNIQUES RECHERCHES (Int. Cl.5) |

Le présent rapport a été établi pour toutes les revendications

| Lieu de la recherche | Date d'achèvement de la recherche | Examinateur |
|---|---|---|
| LA HAYE | 02-07-1992 | RINKEL L.J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)